# NEUE EUROPÄISCHE PATENTSCHRIFT

(11) **EP 3 161 476 B2**
(45) Veröffentlichungstag und Bekanntmachung des Hinweises auf die Entscheidung über den Einspruch: **20.07.2022**
(45) Hinweis auf die Patenterteilung: 15.11.2017
(21) Anmeldenummer: 16704788.5
(22) Anmeldetag: 26.01.2016
(51) Int. Cl.: G01N 33/36, G01D 11/24

(54) **MODULARES GARNPRÜFGERÄT**
MODULAR YARN TESTER
APPAREIL MODULAIRE DE CONTRÔLE DE FIL

(30) Priorität: 20.03.2015 CH 4062015
(43) Veröffentlichungstag der Anmeldung: 03.05.2017
(73) Patentinhaber: Uster Technologies AG, 8610 Uster (CH)
(72) Erfinder: DE VRIES, Loris, 8625 Gossau (CH); FENNER, Jürg, 8600 Dübendorf (CH); KUSTER, Martin, 8733 Eschenbach (CH)
(74) Vertreter: Pliska, Pavel
(86) Internationale Anmeldenummer: PCT/CH2016/000016
(87) Internationale Veröffentlichungsnummer: WO 2016/149838

(56) Entgegenhaltungen:
- EP-A2- 2 641 489
- CH-A5- 671 982
- CH-A5- 671 982
- DE-A1- 2 152 130
- DE-A1- 2 152 130
- GB-A- 2 192 722
- KR-A- 20030 029 767
- US-A- 3 788 138
- US-A- 4 121 450
- US-A- 5 050 437
- US-A1- 2011 193 572
- US-B2- 8 079 255
- "USTER TESTER 5-S800 TECHNICAL DATA", , Dezember 2005 (2005-12), XP55261296, Gefunden im Internet: URL:http://uster.com/fileadmin/customer/Kn owledge/Textile_Know_How/Quality_managemen t/UT5-S800_TechnData-e.pdf [gefunden am 2016-03-29]

## Beschreibung

### FACHGEBIET

Die vorliegende Erfindung liegt auf dem Gebiet der textilen Qualitätsprüfung und bezieht sich auf ein modulares Prüfgerät zur Prüfung von länglichem textilem Prüfgut wie Garn, Vorgarn oder Faserband, gemäss dem Oberbegriff des ersten Patentanspruchs. Das erfindungsgemässe Prüfgerät ist als eigenständige, nicht in einen Produktionsprozess eingebundene Vorrichtung konzipiert und somit im Off-line-Betrieb, bspw. im Textillabor, einsetzbar.

### STAND DER TECHNIK

Prüfgeräte dieser Art werden dort eingesetzt, wo die Qualität von Garnen oder anderen in Faden- oder Bandform vorliegenden textilen Materialien geprüft werden muss. Es geht darum, einerseits eine gleich bleibende Qualität der herzustellenden textilen Produkte zu erreichen, andererseits aber auch darum, den textilen Produktionsprozess anhand von Stichproben off-line zu überwachen.

Geprüft und protokolliert werden mit Prüfgeräten dieser Art in der Regel eine ganze Reihe unterschiedlicher Parameter des zu prüfenden textilen Prüfgutes. Die Anzahl der zu prüfenden Parameter und damit natürlich auch die Komplexität des dazu notwendigen Prüfgerätes hängen von verschiedenen Faktoren ab, so beispielsweise davon, ob Stapel- oder Filamentgarne geprüft werden müssen, aber auch davon, welche Qualitätsstandards für das Textilprodukt vorgegeben sind. Zu den interessierenden Parametern gehört heute in der Regel zumindest die Massenungleichmässigkeit, die mit einem kapazitiven Sensor gemessen wird. Oftmals und gerade bei Naturgarnen kommen aber auch noch optische Sensoren beispielsweise zur Messung von Haarigkeit, Haarigkeitslängen, Fremdstoffe etc. zum Einsatz. Verschiedene weitere interessierende Parameter können dazu treten.

Die bekannten Prüfgeräte dieser Art arbeiten nach dem Durchlaufprinzip, d. h. das zu prüfende Garn wird in einem einzigen Prüfdurchlauf von einer Garnspule abgezogen und durchläuft in serieller Weise im Prüfgerät eine Anordnung von Sensoren, um die zu bestimmenden Parameter zu messen. Dies ist grundsätzlich ein kontinuierlicher Prozess. Je nach Automatisierungsgrad ist auch die mechanische Erfassung und Einführung des Prüfgutes in das Prüfgerät in demselben realisiert. Die Auswertung und statistische Analyse der Sensorsignale erfolgen in der Regel in einer mit dem Prüfgerät verbundenen Steuer- und Auswerteeinheit. Diese interessiert im Kontext der vorliegenden Erfindung jedoch nicht.

Im Zuge der Entwicklung auf diesem Technologiegebiet sind die Anforderungen an Prüfgeräte zur Prüfung von Garnen, Vorgarnen und Faserbändern immer weiter gestiegen. Dies betrifft einerseits den Automatisierungsgrad in der Bereitstellung und in der Handhabung des Prüfgutes, anderseits auch die Zahl der zu messenden bzw. interessierenden Parameter. Bereits ältere Prüfgeräte dieser Art waren in der Regel schon dazu ausgebildet, Grundparameter wie etwa die Massenungleichmässigkeit eines zu prüfenden Garnes im Durchlaufverfahren kontinuierlich zu messen. Erweiterungen liessen sich aber nur mit zusätzlich an- oder einbaubaren separaten Messeinheiten verwirklichen, denn es handelte sich nicht um "offene" Systeme, also nicht um Systeme, die speziell für universelle Erweiterbarkeit konzipiert waren.

Die GB-2'192'722 A und die US-4,845,983 A beschreiben jeweils eine Vorrichtung zur automatischen Bestimmung von Kenngrössen von textilem Prüfgut wie Garnen, Vorgarnen und Bändern im Durchlaufverfahren. Das eigentliche Prüfgerät umfasst eine Messeinheit zur Gleichmässigkeitsprüfung mit einem Messorgan zur Erfassung von Masseschwankungen, eine Führungseinrichtung und eine Vorschubeinrichtung für das Prüfgut. Dazu kann ein weiteres Messorgan für die Erfassung der Haarigkeit des Prüfgutes hinzutreten. Zwar wird in dieser Offenbarung bereits von einer Modularität gesprochen, es handelt sich dabei aber nicht um Modularität im Sinn eines "offenen" Systems, sondern wie oben dargelegt lediglich um bis zu maximal drei in der Messeinheit einbaubaren und in unterschiedlicher Reihenfolge zusammenbaubaren Moduleinheiten. Die Moduleinheiten können zu einer gemeinsam nutzbaren Grundfunktionalität in der Form einer Fadenführungseinrichtung, einer Vorschubeinrichtung und einer Absaugdüse hinzutreten, erfordern ansonsten aber separate elektrische Anschlüsse, separate Kühlungs- und Lüftungseinrichtungen und insbesondere auch separate Befestigungsmittel wie einen Rahmen mit anpassbarem Bügeloberteil. Insbesondere sind auch keine Mittel zur Erhöhung des Automatisierungsgrades in der Bereitstellung und in der Handhabung des Prüfgutes vorhanden. Das Prüfgut muss bei den Vorrichtungen gemäss der GB-2'192'722 A und der US-4,845,983 A manuell eingelegt oder eingeführt werden.

Eine Weiterentwicklung eines derartigen Prüfgerätes ist in der US-2008/0209998 A1 offenbart. Es handelt sich um ein Prüfgerät zur Prüfung von Garnen mit grundsätzlich ähnlichem Aufbau wie denjenigen der GB-2'192'722 A und der US-4,845,983 A. Allerdings sind hier eine automatisierte Einführungsvorrichtung und ein halbautomatischer Garnwechsler bereits integriert. Das zu prüfende Garn ist in einem leicht gekrümmten, im Wesentlichen aber vertikal verlaufenden Prüfgutkanal geführt. Es sind zudem auch Mittel vorhanden, mit denen unerwünschte Partikel wie Staub oder Faserteile aus dem Prüfgutkanal abgesaugt werden können. Auffällig ist der leicht konvex gekrümmte Verlauf des Prüfgutkanals, was der Lagesicherheit des Prüfgutes im Prüfgutkanal dient. Der Garnwechsler gemäss der US-2008/0209998 A1 ist halbautomatisch, was bedeutet, dass die zu prüfenden Garne zwar manuell in den Garnwechsler eingelegt werden müssen, die Position des Garnwechslers im Prüfbetrieb dann aber automatisch auf den Prüfgutkanal und die Einführungsvorrichtung ausrichtbar ist.

Das in der US-2008/0209998 A1 offenbarte Prüfgerät zeigt zudem, dass die automatische Einführungsvorrichtung so angeordnet ist, dass sie im Wesentlichen neben und entlang der Längserstreckung des Prüfgutkanals verläuft und zudem auch vor den Frontplatten der Einzelmodule des Prüfgerätes angebracht ist. Das bedeutet zwar, dass die Garnerfassungs- bzw. Garneinführungsmittel der Einführungsvorrichtung bei einer Garneinführung in das Prüfgerät keine weiten Wege zurücklegen müssen; es bedeutet aber auch, dass das in der US-2008/0209998 A1 offenbarte Prüfgerät ebenfalls kein leicht an geänderte Umstände anpassbares "offenes" System ist, weil nämlich zum Einbau eines weiteren Messmoduls stets die Einführungsvorrichtung ab- und wieder eingebaut werden muss.

Die US-2005/0162837 A1 offenbart eine Vorrichtung zum Befestigen und Lösen einer Leiterplatte. Die Vorrichtung beinhaltet einen zu seiner Front hin offenen Hauptteil mit einer Mehrzahl von Gestellen in einem inneren Abschnitt davon. Ferner beinhaltet die Vorrichtung eine Führungshalterung, in die eine Seite einer Leiterplatte eingeklemmt ist. Die Führungshalterung und die Leiterplatte können von der Front her in eines der Gestelle im Hauptteil eingeschoben werden. Die Führungshalterung weist einen Auswurfmechanismus zum Befestigen und Auswerfen der Leiterplatte auf.

### DARSTELLUNG DER ERFINDUNG

Der Markt fordert Garnprüfgeräte, die den unterschiedlichsten Anforderungen in Bezug auf die Menge messbarer Parameter gerecht werden und die auch bei einer Erweiterung der Menge messbarer Parameter keinen unverhältnismässig grossen Anpassungsaufwand erfordern.

Es ist eine Aufgabe der vorliegenden Erfindung, ein leichter an geänderte Umstände anpassbares modulares Prüfgerät für längliches textiles Prüfgut wie Garn, Vorgarn oder Faserband zur Verfügung zu stellen. Eine weitere Aufgabe besteht darin, eine einfachere Montage, Wartung und Reparatur des Prüfgerätes zu ermöglichen.

Diese und andere Aufgaben werden durch das erfindungsgemässe modulare Prüfgerät, wie es im ersten Patentanspruch definiert ist, gelöst. Vorteilhafte Ausführungsformen sind in den abhängigen Patentansprüchen angegeben.

Das erfindungsgemässe modulare Prüfgerät dient zur Prüfung von länglichem textilem Prüfgut wie Garn, Vorgarn oder Faserband. Das Prüfgerät weist einen entlang einer Front des Prüfgerätes verlaufenden Prüfgutpfad und einen turmartigen Aufbau mit einer Mehrzahl entlang des Prüfgutpfades nacheinander angeordneter Funktionsmodule zur Messung verschiedener Parameter des textilen Prüfgutes auf. Die Funktionsmodule sind als Einschübe ausgebildet. Am turmartigen Aufbau sind Einschubplätze für die Funktionsmodule derart angeordnet, dass jedes der Funktionsmodule von der Front her in einen der Einschubplätze einschiebbar und aus dem Einschubplatz ausschiebbar ist. Jedes der Funktionsmodule ist mittels von der Front her zugänglicher Befestigungsmittel in einem der Einschubplätze lösbar befestigbar. Ferner weist das modulare Prüfgerät eine Grundinfrastruktur zur Bereitstellung einer Grundfunktionalität auf. Der turmartige Aufbau umfasst ein Basisgehäuse für die Grundinfrastruktur und ein auf das Basisgehäuse aufgesetztes Optionengehäuse für optionale Funktionsmodule. Sowohl das Basisgehäuse als auch das Optionengehäuse weisen jeweils mehrere Einschubplätze für die Funktionsmodule auf. Die Aufteilung in Basisgehäuse und Optionengehäuse erlaubt eine bessere Anpassbarkeit an bestehende und künftige Marktbedürfhisse. Die optionalen Funktionsmodule sind vorzugsweise in einem der Einschubplätze im Optionengehäuse wahlfrei positionierbar. Wird beispielsweise nur ein optionales Funktionsmodul gewünscht, so können die restlichen Einschubplätze im Optionengehäuse mit Blindmodulen versehen sein.

Unter dem Begriff "Grundfunktionalität" versteht man dabei solche Funktionen, die selbst einfachste Prüfgeräte zur Prüfung von länglichem textilem Prüfgut sinnvollerweise mindestens aufweisen sollten, um nutzbringend eingesetzt werden zu können. Die Grundinfrastruktur zur Bereitstellung der Grundfunktionalität kann bspw. mindestens eine Fördereinrichtung für das textile Prüfgut, eine Absaugöffnung für das textile Prüfgut und einen Sensor zur Gleichmässigkeitsprüfung des textilen Prüfgutes umfassen.

In einer Ausführungsform weist mindestens eines der Funktionsmodule eine Frontplatte auf, welche das Funktionsmodul trägt und gegen die Front hin abdeckt. Das Funktionsmodul kann mit von der Front her zugänglichen und lösbaren Befestigungsmitteln, bspw. in der Form von Schrauben, am turmartigen Aufbau befestigbar sein. An der Frontplatte sind vorzugsweise ein Sensor einerseits und eine Wanne, welche weitere Elemente des Funktionsmoduls trägt, andererseits befestigt. Eines der weiteren von der Wanne getragenen Elemente des Funktionsmoduls ist z. B. eine Modulleiterplatte mit einer elektronischen Schaltung zum Ansteuern des Sensors und zur Auswertung der vom optischen Sensor detektierten Signale.

In einer Ausführungsform weist das modulare Prüfgeräteine automatische Einführungsvorrichtung zum Einführen des textilen Prüfgutes in den Prüfgutpfad auf, welche derart am turmartigen Aufbau angeordnet ist, dass sie das Einschieben und Ausschieben der Funktionsmodule nicht behindert. Die automatische Einführungsvorrichtung ist vorzugsweise an einer Seite des Prüfgerätes, die von der Front verschieden ist, angeordnet.

Mit dem erfindungsgemässen modularen Prüfgerät erreicht man eine im Vergleich zu den bekannten Lösungen echte oder zumindest stark verbesserte Modularität, wenn der Begriff so verstanden wird, dass ein Austausch eines bereits vorhandenen Moduls oder auch ein Hinzufügen eines bisher nicht vorhandenen Moduls wesentlich erleichtert ist. Es handelt sich somit um eine Lösung, die man als "offen" bezeichnen kann, weil sie speziell für universelle Erweiterbarkeit konzipiert ist. Umbauten sind wesentlich erleichtert und können in kürzerer Zeit durchgeführt werden.

### AUFZÄHLUNG DER ZEICHNUNGEN

Nachfolgend werden Ausführungsformen der Erfindung anhand der Zeichnungen detailliert erläutert.
- Figur 1: zeigt ein erfindungsgemässes modulares Prüfgerät in fertigem Zustand.
- Figur 2: zeigt ein erfindungsgemässes modulares Prüfgerät mit einem Basisgehäuse und einem Optionengehäuse in halbfertigem Zustand mit eingesetzten Funktionsmodulen.
- Figur 3: zeigt das modulare Prüfgerät von Fig. 2 mit teilweise ausgezogenen Funktionsmodulen.
- Figur 4: zeigt das modulare Prüfgerät von Fig. 2 in vormontiertem Zustand.
- Figur 5: zeigt ein Funktionsmodul des modularen Prüfgerätes von Fig. 2.
- Figur 6: zeigt das Basisgehäuse des modularen Prüfgerätes von Fig. 2 in vormontiertem Zustand.

### AUSFÜHRUNG DER ERFINDUNG

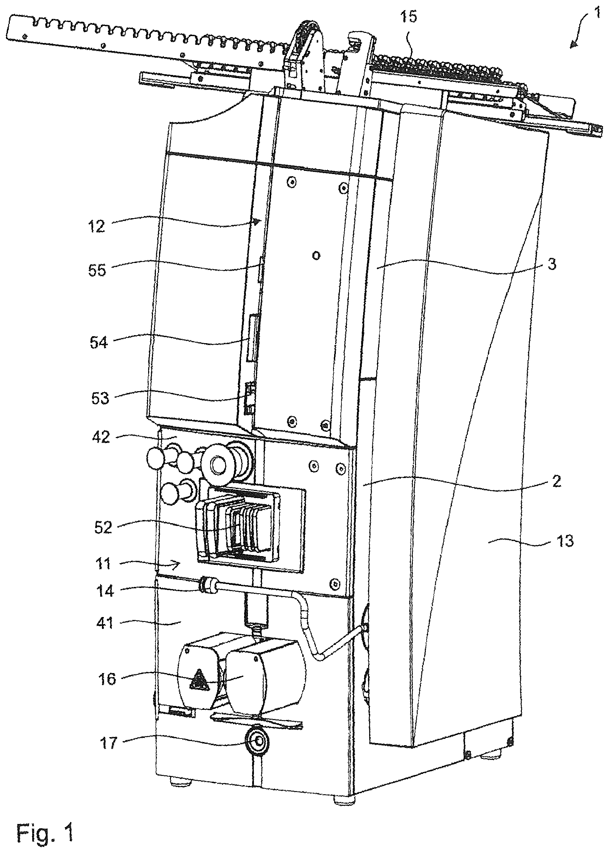

**Figur 1** zeigt beispielhaft eine Ausführungsform des erfindungsgemässen modularen Prüfgerätes 1. Das Prüfgerät 1 weist eine Front 11 auf, entlang welcher ein Prüfgutpfad 12 für ein zu prüfendes (nicht eingezeichnetes) textiles Prüfgut verläuft. Auf einer Seite des Prüfgerätes 1, welche von der Front 11 verschieden ist, ist eine automatische Einführungsvorrichtung 13 zum Einführen des Prüfgutes in den Prüfgutpfad 12 angebracht. Zum Einbringen in den Prüfgutpfad 12 wird das Prüfgut von einem verschiebbaren und drehbaren Greifer 14 der Einführungsvorrichtung 13 ergriffen und durch eine entsprechende Bewegung des Greifers 14 eingebracht.

Während des Prüfvorgangs tritt das Prüfgut durch eine automatische Garnwechselvorrichtung 15 in den Prüfgutpfad 12 ein. Im Prüfgutpfad 12 durchläuft das Prüfgut verschiedene Sensoren 52-55, auf die weiter unten eingegangen wird. Das Prüfgut wird von einer Fördereinrichtung 16 entlang seiner Längsrichtung durch den Prüfgutpfad 12 gefördert. Die Fördereinrichtung 16 kann z. B. als Rollenlieferwerk mit zwei zusammenwirkenden Förderrollen von denen mindestens eine zur Rotation angetrieben ist, ausgebildet sein. Schliesslich verlässt das Prüfgut den Prüfgutpfad 12 durch eine Absaugöffnung 17.

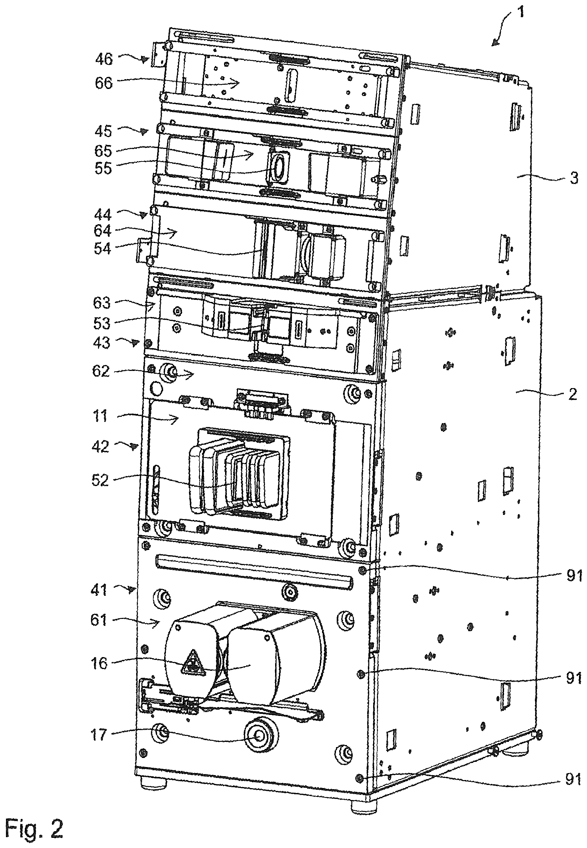

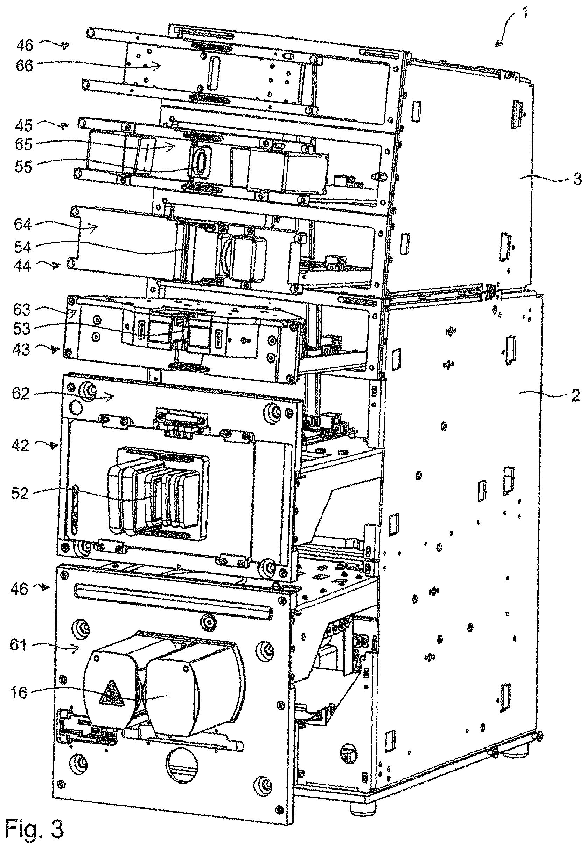

In den **Figuren 2-4** ist das Prüfgerät 1 von Figur 1 in halbfertigem bzw. in vormontiertem Zustand dargestellt. Gegenüber dem fertigen Zustand von Figur 1 fehlen hier die Einführungsvorrichtung 13, die Garnwechselvorrichtung 15 und verschiedene Abdeckungen, besonders Abdeckungen der Front 11. Zur Illustration der Erfindung eignet sich diese Darstellung besser als diejenige von Figur 1, weil sie viele erfindungswesentliche Elemente besser zeigt. Das Prüfgerät 1 hat einen turmartigen Aufbau mit einem Basisgehäuse 2 und einem darauf aufgesetzten Optionengehäuse 3. Das Basisgehäuse 2 und das Optionengehäuse 3 weisen Einschubplätze 71-76 auf, in welche Funktionsmodule 41-46 zur Prüfung des länglichen Prüfgutes eingeschoben sind.

Ein im Basisgehäuse 2 eingebautes erstes Funktionsmodul 41 beinhaltet im vorliegenden Fall die Fördereinrichtung 16 und die Absaugöffnung 17. Ein zweites Funktionsmodul 42 beinhaltet mindestens einen kapazitiven Sensor 52 zur Gleichmässigkeitsprüfung des Prüfgutes. Ein drittes Funktionsmodul 43 beinhaltet einen optischen Mehrzwecksensor 53. An dieser Stelle könnte ebenso gut auch ein anderes der optional einsetzbaren Funktionsmodule 43-46 eingefügt sein.

Auf das Basisgehäuse 2 aufgesetzt und fest mit demselben verbunden ist das Optionengehäuse 3. Das Optionengehäuse 3 bietet im vorliegenden Ausführungsbeispiel Platz für maximal drei Funktionsmodule 44-46, von denen jedes die gleiche Grösse hat wie das im Basisgehäuse 2 eingeschobene dritte Funktionsmodul 43. Das ermöglicht eine wahlfreie Platzierung der optionalen Funktionsmodule 43-46, selbstverständlich nur innerhalb einer messtechnisch sinnvollen Reihenfolge. Ein viertes Funktionsmodul 44 beinhaltet einen optischen Sensor 54 zur Detektion von Verunreinigungen in dem Prüfgut. Ein fünftes Funktionsmodul 45 beinhaltet einen optischen Sensor 55 zur Prüfung der Haarigkeit des Prüfgutes. Ein sechstes Funktionsmodul 46 ist im vorliegenden Ausführungsbeispiel ein Blindmodul, also ein leerer Einschub, der lediglich Platzhalterfunktion hat.

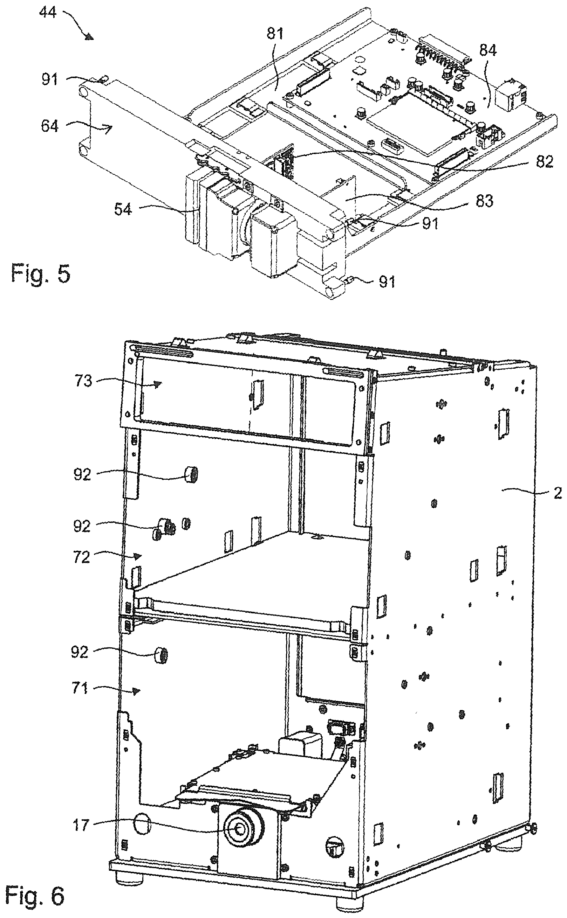

Jedes Funktionsmodul 41-46 weist eine Frontplatte 61-66 auf, welche das Funktionsmodul 41-46 gegen die Front 11 hin abdeckt. Die Frontplatte 61-66 ist das tragende Element für das entsprechende Funktionsmodul 41-46. Der Rest des Funktionsmoduls 41-46 ist also an der Frontplatte 61-66 befestigt und wird von dieser getragen. Daher muss jede Frontplatte 61-66 derart fest und starr ausgebildet sein, dass sie das jeweilige Funktionsmodul 41-46 tragen kann. Die Frontplatten 61-66 aller Funktionsmodule 41-46 sind mit von der Front 11 her zugänglichen und lösbaren Befestigungsmitteln 91, bspw. in der Form von Schrauben, Einrastverbindungen etc., am Basisgehäuse 2 bzw. am Optionengehäuse 3 befestigbar. Beispielhaft eingezeichnet ist dies in der Figur 2 am ersten Funktionsmodul 41 und in der Figur 5 am vierten Funktionsmodul 44.

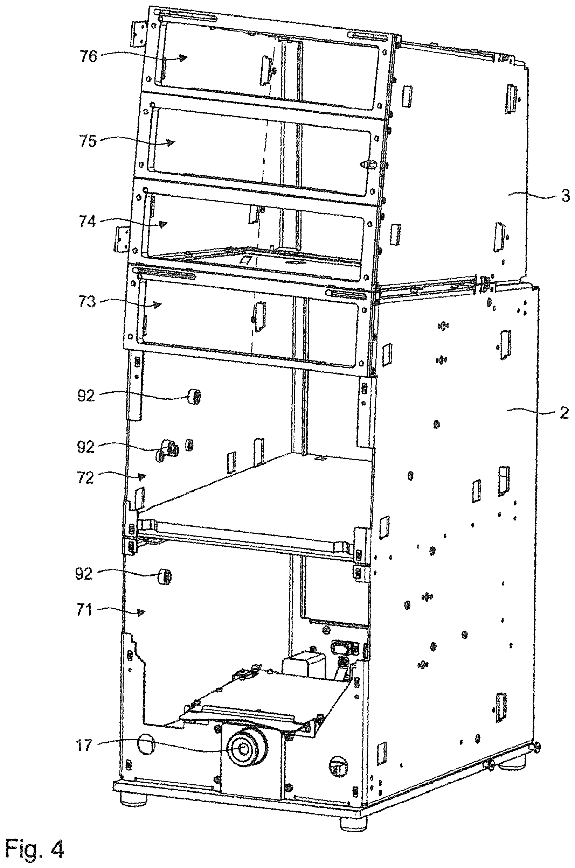

Die Einschubplätze 71-76 für die Einschübe oder Funktionsmodule 41-46 können je nach Art des Funktionsmoduls unterschiedlich ausgestaltet sein. So können beispielsweise für grosse und schwere Funktionsmodule wie das erste Funktionsmodul 41 und das zweite Funktionsmodul 42 Zapfen 92 (siehe Fig. 4) als Führungs- und Haltemittel vorhanden sein. Die Zapfen 92 erleichtern die Handhabung der betreffenden Funktionsmodule 41, 42 durch das Wartungspersonal, also das Einführen und Entfernen der Funktionsmodule 41, 42 in das bzw. aus dem Basisgehäuse 1. Kleinere und leichtere Funktionsmodule wie die Funktionsmodule 43-46 brauchen derartige Zapfen meist nicht und werden nach dem Einschieben allein von den von der Front 11 her zugänglichen Befestigungsmitteln 91 gehalten. Dies macht den Aufbau, die Montage und die Wartung des Prüfgerätes 1 besonders einfach.

Ein Funktionsmodul 44 des erfindungsgemässen Prüfgerätes 1 ist in **Figur 5** dargestellt, z. B. das vierte Funktionsmodul 44 mit dem optischen Sensor 54 zur Detektion von Verunreinigungen in dem Prüfgut. Die fest und starr ausgebildete Frontplatte 64 trägt das ganze Funktionsmodul 44 und dient zu seiner Befestigung am Optionengehäuse 3 mit vier von der Front 11 her zugänglichen und lösbaren Schrauben 91. Aussen an der Frontplatte 64 ist der optische Sensor 54 angebracht, der z. B. eine Beleuchtungseinheit, eine Detektionseinheit und weitere optische Komponenten beinhalten kann. Der optische Sensor 54 kann auch eine Beleuchtungsleiterplatte 82 mit einer Beleuchtungsschaltung zum Ansteuern der Beleuchtungseinheit sowie eine Detektionsleiterplatte 83 mit einer Detektionsschaltung zum Ansteuern der Detektionseinheit und zur Vorverarbeitung der detektierten Signale beinhalten. Innen an der Frontplatte 64 ist eine Wanne 81, z. B. aus einem Metallblech, befestigt. Die Wanne 81 ist im Wesentlichen horizontal, d. h. rechtwinklig zur Ebene der Frontplatte 64, angeordnet. Dank ihrer Befestigung an der Frontplatte 64 bedarf sie keiner weiteren Befestigung oder Stützung am Optionengehäuse 3. Sie trägt z. B. eine Modulleiterplatte 84 mit einer elektronischen Schaltung zum Ansteuern des optischen Sensors 54 und zur Auswertung der vom optischen Sensor 54 detektierten Signale. Die Wanne 81 kann ferner weitere Elemente wie z. B. (nicht eingezeichnete) Kabel zur Verbindung der Modulleiterplatte 84 mit der Beleuchtungsleiterplatte 82 bzw. der Detektionsleiterplatte 83 tragen. Die übrigen optionalen Funktionsmodule 43, 45, 46 können ähnlich aufgebaut sein wie das hier beispielhaft diskutierte vierte Funktionsmodul 44.

**Figur 6** zeigt ein alleinstehendes Basisgehäuse 2 in derselben Ansicht wie Figur 4. Die Figur 6 soll veranschaulichen, dass das Basisgehäuse 2 mit den eingesetzten Funktionsmodulen 41-43 als eigenständiges Prüfgerät 1 eingesetzt werden kann. Beim Optionengehäuse 3, wie es in den Figuren 1-4 eingezeichnet ist, handelt es sich also um eine Erweiterung des Prüfgerätes 1.

Selbstverständlich ist die vorliegende Erfindung nicht auf die oben diskutierten Ausführungsformen beschränkt. Bei Kenntnis der Erfindung wird der Fachmann weitere Varianten innerhalb des Schutzumfangs der Ansprüche herleiten können, die auch zum Gegenstand der vorliegenden Erfindung gehören.

### BEZUGSZEICHENLISTE

- 1: Prüfgerät
- 11: Front
- 12: Prüfgutpfad
- 13: Einführungsvorrichtung
- 14: Greifer
- 15: Garnwechselvorrichtung
- 16: Fördereinrichtung
- 17: Absaugöffnung

- 2: Basisgehäuse

- 3: Optionengehäuse

- 41-46: Funktionsmodule

- 52: kapazitive Massesensoren
- 53: optischer Mehrzwecksensor
- 54: optischer Sensor zur Detektion von Verunreinigungen
- 55: optischer Sensor zur Prüfung der Haarigkeit

- 61-66: Frontplatten

- 71-76: Einschubplätze

- 81: Wanne
- 82: Beleuchtungsleiterplatte
- 83: Detektionsleiterplatte
- 84: Modulleiterplatte

- 91: Befestigungsmittel
- 92: Zapfen

## Patentansprüche

1. Modulares Prüfgerät (1) zur Prüfung von länglichem textilem Prüfgut wie Garn, Vorgarn oder Faserband, wobei
das Prüfgerät (1) einen entlang einer Front (11) des Prüfgerätes (1) verlaufenden Prüfgutpfad (12) und einen turmartigen Aufbau mit einer Mehrzahl entlang des Prüfgutpfades (12) nacheinander angeordneter Funktionsmodule (41-46) aufweist, die Funktionsmodule (41-46) als Einschübe ausgebildet sind,
am turmartigen Aufbau Einschubplätze (71-76) für die Funktionsmodule (41-46) derart angeordnet sind, dass jedes der Funktionsmodule (41-46) von der Front (11) her in einen der Einschubplätze einschiebbar und aus dem Einschubplatz (71-76) ausschiebbar ist,
jedes der Funktionsmodule (41-46) mittels von der Front (11) her zugänglicher Befestigungsmittel (91) in einem der Einschubplätze (71-76) lösbar befestigbar ist, das Prüfgerät (1) eine Grundinfrastruktur zur Bereitstellung einer Grundfunktionalität aufweist und
der turmartige Aufbau ein Basisgehäuse (2) für die Grundinfrastruktur umfasst,
**dadurch gekennzeichnet, dass**
die Funktionsmodule (41-46) zur Messung verschiedener Parameter des textilen Prüfgutes ausgebildet sind,
der turmartige Aufbau ein auf das Basisgehäuse (2) aufgesetztes Optionengehäuse (3) für optionale Funktionsmodule (43-46) umfasst und
sowohl das Basisgehäuse (2) als auch das Optionengehäuse (3) jeweils mehrere Einschubplätze (71-76) für die Funktionsmodule (41-46) aufweisen.

2. Modulares Prüfgerät (1) nach Anspruch 1, wobei die optionalen Funktionsmodule (43-46) in einem der Einschubplätze (74-76) im Optionengehäuse (3) wahlfrei positionierbar sind.

3. Modulares Prüfgerät (1) nach Anspruch 1 oder 2, wobei die Grundinfrastruktur zur Bereitstellung der Grundfunktionalität mindestens eine Fördereinrichtung (16) für das textile Prüfgut, eine Absaugöffnung (17) für das textile Prüfgut und einen Sensor (52) zur Gleichmässigkeitsprüfung des textilen Prüfgutes umfasst.

4. Modulares Prüfgerät (1) nach einem der vorangehenden Ansprüche, wobei mindestens eines (44) der Funktionsmodule (41-46) eine Frontplatte (64) aufweist, welche das Funktionsmodul (44) trägt und gegen die Front (11) hin abdeckt.

5. Modulares Prüfgerät (1) nach Anspruch 4, wobei das Funktionsmodul (44) mit von der Front (11) her zugänglichen und lösbaren Befestigungsmitteln (91), bspw. in der Form von Schrauben, am turmartigen Aufbau befestigbar ist.

6. Modulares Prüfgerät (1) nach Anspruch 4 oder 5, wobei an der Frontplatte (64) ein Sensor (54) einerseits und eine Wanne (81), welche weitere Elemente des Funktionsmoduls (44) trägt, andererseits befestigt sind.

7. Modulares Prüfgerät nach Anspruch 6, wobei eines der weiteren von der Wanne (81) getragenen Elemente des Funktionsmoduls (44) eine Modulleiterplatte (84) mit einer elektronischen Schaltung zum Ansteuern des Sensors (54) und zur Auswertung der vom optischen Sensor (54) detektierten Signale ist.

8. Modulares Prüfgerät (1) nach einem der vorangehenden Ansprüche, wobei das Prüfgerät (1) eine automatische Einführungsvorrichtung (13) zum Einführen des textilen Prüfgutes in den Prüfgutpfad (12) aufweist, welche derart am turmartigen Aufbau angeordnet ist, dass sie das Einschieben und Ausschieben der Funktionsmodule (41-46) nicht behindert.

9. Modulares Prüfgerät (1) nach Anspruch 8, wobei die automatische Einführungsvorrichtung (13) an einer Seite des Prüfgerätes (1), die von der Front (11) verschieden ist, angeordnet ist.

## Claims

1. A modular tester (1) for testing elongated textile test material such as yarn, roving or sliver, wherein
the tester (1) comprises a test material path (12) extending along a front (11) of the tester (1) and a turret-like setup with a plurality of functional modules (41 to 46) which are arranged in succession along the test material path (12),
the functional modules (41 to 46) are formed as slide-in modules,
slots (71 to 76) for the functional modules (41 to 46) are arranged on the turret-like setup in such a way that each of the functional modules (41 to 46) is insertable into one of the slots from the front (11) and is slidable out of the slot (71 to 76),
each of the functional modules (41 to 46) is detachably fastenable in one of the slots (71 to 76) by means of fastening means (91) which are accessible from the front (11), the test device (1) comprises a basic infrastructure for providing a basic functionality, and
the turret-like setup comprises a base housing (2) for the basic infrastructure,
**characterized in that**
the functional modules (41 to 46) are configured for measuring various parameters of the textile test material,
the turret-like setup comprises an optional housing (3) for optional functional modules (43 to 46), said optional housing (3) being positioned on the base housing (2), and
both the base housing (2) and also the optional housing (3) each comprise several slots (71 to 76) for the functional modules (41 to 46).

2. A modular tester (1) according to claim 1, wherein the optional functional modules (43 to 46) are electively positionable in one of the slots (74 to 76) in the optional housing (3).

3. A modular tester (1) according to claim 1 or 2, wherein the basic infrastructure for providing the basic functionality comprises at least one conveying device (16) for the textile test material, a suction opening (17) for the textile test material and a sensor (52) for testing the uniformity of the textile test material.

4. A modular tester (1) according to one of the preceding claims, wherein at least one (44) of the functional modules (41 to 46) comprises a front panel (64) which carries the functional module (44) and covers it against the front (11).

5. A modular tester (1) according to claim 4, wherein the functional module (44) can be fastened to the turret-like setup by means of fastening means (91), e.g. in form of screws, which are accessible and releasable from the front (11).

6. A modular tester (1) according to claim 4 or 5, wherein a sensor (54) is fastened to the front panel (64) on the one hand and a trough (81) which carries further elements of the functional module (44) is fastened to the front panel (64) on the other hand.

7. A modular tester (1) according to claim 6, wherein one of the further elements of the functional module (44) which is carried by the trough (81) is a modular circuit board (84) with an electronic circuit for controlling the sensor (54) and for evaluating the signals detected by the optical sensor (54).

8. A modular tester (1) according to one of the preceding claims, wherein the tester (1) comprises an automatic insertion apparatus (13) for inserting the textile test material into the test material path (12), which insertion apparatus is arranged on the turret-like setup in such a way that it does not obstruct the insertion and removal of the functional modules (41 to 46).

9. A modular tester (1) according to claim 8, wherein the automatic insertion apparatus (13) is arranged on one side of the tester (1) which differs from the front (11).

## Revendications

1. Appareil de contrôle modulaire (1) pour le contrôle de matière textile allongée à contrôler telle qu'un fil, une mèche ou un ruban de fibres, dans lequel l'appareil de contrôle (1) comprend un trajet de la matière à contrôler (12) passant le long d'une face avant (11) de l'appareil de contrôle (1) et une superstructure en forme de tour avec plusieurs modules fonctionnels (41-46) disposés les uns à la suite des autres sur le trajet de la matière à contrôler (12),
les modules fonctionnels (41-46) sont conformés comme des tiroirs,
des emplacements d'insertion (71-76) pour les modules fonctionnels (41-46) sont disposés sur la superstructure en forme de tour de telle manière que chacun des modules fonctionnels (41-46) puisse être inséré dans l'un des emplacements d'insertion et extrait de l'emplacement d'insertion (71-76) en passant par la face avant (11),
chacun des modules fonctionnels (41-46) est fixé de manière amovible dans l'un des emplacements d'insertion (71-76) au moyen de moyens de fixation (91) accessibles par la face avant (11),
l'appareil de contrôle (1) présente une infrastructure de base assurant une fonction de base et
la superstructure en forme de tour comprend un boîtier de base (2) pour l'infrastructure de base,
**caractérisé en ce que**
les modules fonctionnels (41-46) sont configurés pour mesurer différents paramètres de la matière à contrôler textile,
la superstructure en forme de tour comprend un boîtier d'options (3) posé sur le boîtier de base (2) pour des modules fonctionnels en option (43-46) et le boîtier de base (2) et le boîtier d'options (3) présentent chacun plusieurs emplacements d'insertion (71-76) pour les modules fonctionnels (41-46).

2. Appareil de contrôle modulaire (1) selon la revendication 1, dans lequel les modules fonctionnels (43-46) en option peuvent être positionnés au choix dans l'un des emplacements d'insertion (74-76) du boîtier d'options (3).

3. Appareil de contrôle modulaire (1) selon la revendication 1 ou 2, dans lequel l'infrastructure de base comprend, pour réaliser la fonction de base, au moins une installation de transport (16) pour la matière textile à contrôler, une ouverture d'aspiration (17) pour la matière textile à contrôler et un capteur (52) pour le contrôle d'uniformité de la matière textile à contrôler.

4. Appareil de contrôle modulaire (1) selon l'une des revendications précédentes, dans lequel au moins un (44) des modules fonctionnels (41-46) présente un panneau de façade (64) qui porte le module fonctionnel (44) et le couvre par rapport à la face avant (11).

5. Appareil de contrôle modulaire (1) selon la revendication 4, dans lequel le module fonctionnel (44) peut être fixé sur la superstructure en forme de tour au moyen de moyens de fixation (91) accessibles par la face avant (11) et pouvant être défaits, par exemple de vis.

6. Appareil de contrôle modulaire (1) selon la revendication 4 ou 5, dans lequel sont fixés au panneau de façade (64), d'une part, un capteur (54) et, d'autre part, une cuvette (81) qui porte d'autres éléments du module fonctionnel (44).

7. Appareil de contrôle modulaire selon la revendication 6, dans lequel l'un des autres éléments du module fonctionnel (44) portés par la cuvette (81) est une carte de circuits intégrés de module (84) avec un circuit électronique pour la commande du capteur (54) et l'analyse des signaux détectés par le capteur optique (54).

8. Appareil de contrôle modulaire (1) selon l'une des revendications précédentes, dans lequel l'appareil de contrôle (1) présente un dispositif d'insertion automatique (13) pour l'insertion de la matière textile à contrôler dans le trajet de la matière à contrôler (12), qui est disposé sur la superstructure en forme de tour de façon à ne pas gêner l'insertion ni l'extraction des modules fonctionnels (41-46).

9. Appareil de contrôle modulaire (1) selon la revendication 8, dans lequel le dispositif d'insertion automatique (13) est disposé sur un côté de l'appareil de contrôle (1) différent de la face avant (11).
